# EUROPÄISCHE PATENTSCHRIFT

(11) **EP 2 710 970 B1**
(45) Veröffentlichungstag und Bekanntmachung des Hinweises auf die Patenterteilung: **17.10.2018**
(21) Anmeldenummer: 13192484.7
(22) Anmeldetag: 04.11.2008
(51) Int. Cl.: A61B 17/32, A61B 17/3203, A61M 5/14

(54) **Wasserstrahlchirurgiegerät**
Water jet surgical device
Appareil chirurgical à jet d'eau

(30) Priorität: 06.11.2007 DE 102007052805
(43) Veröffentlichungstag der Anmeldung: 26.03.2014
(62) Teilanmeldung aus: 08848108.0
(73) Patentinhaber: Erbe Elektromedizin GmbH, 72072 Tübingen (DE)
(72) Erfinder: Wahl, Hans-Jürgen, 72818 Trochtelfingen (DE); Pfäffle, Alexander, 72810 Gomaringen (DE); Kühner, Ralf, 70567 Stuttgart (DE)
(74) Vertreter: Bohnenberger, Johannes

(56) Entgegenhaltungen:
- EP-A- 0 879 578
- US-A- 4 655 742
- US-A- 5 536 242
- US-A1- 2005 059 924
- US-A1- 2006 156 875
- US-A1- 2006 247 743

## Beschreibung

EP-A-879578 offenbart ein Thrombektomievorrichtung, umfassend eine Flüssigkeitsfördereinrichtung, die zur Abgabe einer Flüssigkeit in eine Anschlussleitung eines Instruments mit einer Auslassdüse durch Ansteuersignale aus einer Steuereinrichtung ansteuerbar ist, wobei mindestens eine Messeinrichtung vorgesehen ist.

Die Erfindung betrifft ein Wasserstrahlchirurgiegerät nach dem Oberbegriff des Patentanspruches 1.
Wasserstrahlchirurgiegeräte, mittels derer Gewebe durch einen Hochdruck-Wasserstrahl getrennt werden kann, sind allgemein bekannt. Wenn man ein solches Gerät in der offenen Chirurgie benutzt, so ist das Abführen der "Schneidflüssigkeit" unproblematisch. Bei Verwendung derartiger Geräte bei endoskopischen Operationen, also innerhalb von Körperhöhlen, können durch die Zufuhr der Schneidflüssigkeit Probleme auftreten.
Der Erfindung liegt die Aufgabe zugrunde, ein Wasserstrahlchirurgiegerät der eingangs genannten Art dahingehend aufzuzeigen, dass die insbesondere bei endoskopischen Operationen auftretenden Probleme vermindert werden.
Diese Aufgabe wird durch ein Wasserstrahlchirurgiegerät nach Anspruch 1 gelöst. Insbesondere wird diese Aufgabe durch ein Wasserstrahlchirurgiegerät gelöst, umfassend eine Flüssigkeitsfördereinrichtung, die zur Abgabe einer Flüssigkeit in einer Anschlussleitung eines Chirurgieinstruments mit einer Auslassdüse durch Ansteuersignale aus einer Steuereinrichtung ansteuerbar ist, wobei mindestens eine Messeinrichtung vorgesehen und derart ausgebildet ist, dass nach einem Anschließen des Chirurgieinstruments an die Flüssigkeitsfördereinrichtung die Messeinrichtung Messsignale zur Darstellung einer Menge abgegebener Flüssigkeit in einer Anzeige- oder Registriereinheit erzeugt. Dadurch ist es möglich, bei einer Operation die in eine Körperhöhle eingeführte Schneidflüssigkeit ihrer Menge nach zu bestimmen und mit der Flüssigkeitsmenge zu vergleichen, die vom Operationsfeld abgesaugt wird. Darüber hinaus ist es möglich, eine exakte Planung der Operation vorzunehmen und zwar hinsichtlich der verbrauchten Menge an Schneidflüssigkeit, die in einem Vorratsbehälter vorgehalten wird.

Die Messeinrichtung umfasst vorzugsweise einen Füllsensor und erzeugt ein Füllsignal dann, wenn die Anschlussleitung des Chirurgieinstruments im Wesentlichen bis zur Auslassdüse gefüllt ist und gibt eine zum Füllen der Anschlussleitung benötigte Füllmenge wieder. Auf diese Weise kann das Wasserstrahlchirurgiegerät in dem Sinne funktionsbereit gemacht werden, dass der Operateur dann, wenn er ein Startsignal zum Beginn eines Schneidvorgangs gibt, tatsächlich Schneidflüssigkeit an der Auslassdüse ansteht.

Vorzugsweise ist die Anzeige- oder Registriereinheit derart ausgebildet, dass die Menge abgegebener Flüssigkeit abzüglich der Füllmenge angezeigt wird. Diese Füllmenge kann besonders bei langen Anschlussleitungen oder Chirurgieinstrumenten mit einer hohen Flüssigkeitskapazität relativ beträchtlich sein, sodass die Messung der in den Körper abgegebenen Flüssigkeitsmenge verfälscht wird.

Vorzugsweise ist eine Eingabevorrichtung vorgesehen, über welche die Steuereinrichtung ein dem angeschlossenen Chirurgieinstrument entsprechendes Mengensignal zur Abgabe einer von der Flüssigkeitsfördereinrichtung abzugebenden Flüssigkeitsmenge zuführbar ist. Bei dieser Ausführungsform der Erfindung muss also nicht unbedingt gemessen werden, wann die Anschlussleitung bzw. das Chirurgieinstrument befüllt ist, es wird hier vielmehr eine Menge von Schneidflüssigkeit vorgegeben, die von der Flüssigkeitsfördereinrichtung abgegeben wird, sodass man sich dann sicher sein kann, dass das Gerät befüllt ist.

Diese Eingabe der Flüssigkeitsmenge kann durch eine manuell bedienbare Eingabevorrichtung vorgegeben werden. Bei einer anderen bevorzugten Ausführungsform der Erfindung wird die zum Befüllen des Chirurgieinstrumentes bzw. seiner Anschlussleitung notwendige Flüssigkeitsmenge direkt durch eine Codiereinrichtung im Chirurgieinstrument der Eingabevorrichtung übermittelt, sodass ein manuelles Programmieren entfällt.

Wenn ein Füllsensor vorhanden ist, so kann dieser als Drucksensor ausgebildet sein, der bei einer vorbestimmten Druckänderung oder einem vorbestimmten zeitlichen Verlauf des Druckes das Füllsignal erzeugt. Der Druck der Schneidflüssigkeit ist nämlich niedrig, solange aus der Auslassdüse Luft herausgedrückt wird.

Bei einer anderen Ausführungsform der Erfindung umfasst die Sensoreinrichtung einen Feuchtigkeits- oder Leitfähigkeitssensor, der vorzugsweise sehr nahe an der Auslassdüse angebracht ist und dann ein Signal erzeugt, wenn die Flüssigkeit tatsächlich an diesem Ort angekommen ist.

Ein Verfahren zum Betreiben eines Wasserstrahlchirurgiegerätes kann die folgenden Schritte umfassen:
- Anschließen eines Chirurgieinstruments an das Wasserstrahlchirurgiegerät;
- Erzeugen von Ansteuersignalen zum Ansteuern einer Flüssigkeitsfördereinrichtung derart, dass diese Flüssigkeit solange dem Chirurgieinstrument zuführt, bis dieses im Wesentlichen bis zu seiner Auslassdüse gefüllt ist;
- Beenden der Ansteuersignale und Erzeugen eines Betriebsfreigabesignals derart, dass eine Ansteuerung der Flüssigkeitsfördereinrichtung zum Operationsbetrieb des Wasserstrahlchirurgiegeräts zugelassen wird.

Es wird also in diesem Fall eine Betätigung des Wasserstrahlchirurgiegeräts unter Operationsbedingungen verhindert, bis das Chirurgieinstrument wirklich "startklar" ist.

Nachfolgend wird eine Ausführungsform der Erfindung anhand von Abbildungen näher erläutert. Hierbei zeigen
- - Fig. 1: eine schematische Darstellung zur Erläuterung der verschiedenen Baugruppen und
- - Fig. 2: ein Diagramm zur Erläuterung des Druckverlaufes beim Befüllen des Chirurgieinstruments.

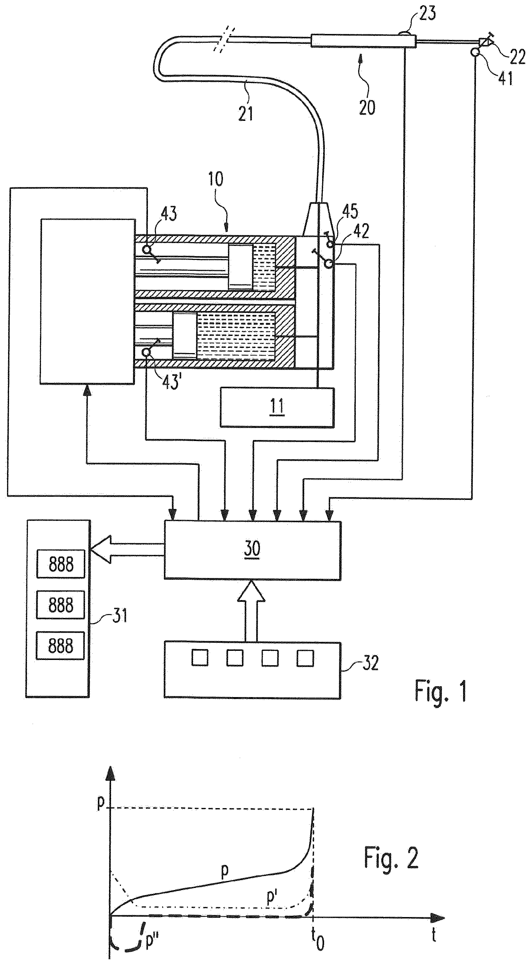

In Fig. 1 ist als Flüssigkeitsfördereinrichtung 10 eine Pumpe mit zwei Kolben dargestellt, die aus einem Vorratsbehälter 11 Schneidflüssigkeit über eine Anschlussleitung 21 eines Chirurgieinstrumentes 20 bis zu einer Auslassdüse 22 fördert, aus welcher bei Betätigung einer Bedienungstaste 23 Schneidflüssigkeit aus der Auslassdüse 22 zum Trennen von Gewebe oder auch zum Injizieren in ein Gewebe ausgestoßen wird. Selbstverständlich sind auch andere Druckerzeugungseinrichtungen verwendbar.

Die Kolben der bei diesem Ausführungsbeispiel gezeigten Flüssigkeitsfördereinrichtung werden hinsichtlich ihrer Position über Positionssensoren 43, 43' überwacht, deren Ausgangssignale einer Steuereinrichtung 30 zugeführt werden.

Weiterhin ist an der Flüssigkeitsfördereinrichtung 10 ein Ankoppelfühler 45 vorgesehen, der dann anspricht, wenn ein Chirurgieinstrument 20 mit der Flüssigkeitsfördereinrichtung 10 verbunden wird.

Der von der Flüssigkeitsfördereinrichtung 10 erzeugte Druck wird mittels eines Drucksensors 42 abgetastet, dessen Ausgangssignale der Steuereinrichtung 30 zugeführt werden. Alternativ oder zusätzlich kann ein Feuchtesensor 41 in der Auslassdüse 22 oder zumindest in deren Nähe vorgesehen sein, der dann anspricht, wenn Schneidflüssigkeit bis zu diesem Punkt gelangt ist.

Die Steuereinrichtung 30 ist mit einer Anzeigeeinheit 31 verbunden, auf welcher die verschiedenen Parameter, insbesondere aber die von der Flüssigkeitsfördereinrichtung 10 geförderte und aus der Auslassdüse 22 ausgestoßene Flüssigkeitsmenge angezeigt wird.

Weiterhin ist eine Eingabevorrichtung 32 vorgesehen, über welche der Steuereinrichtung 30 Betriebsdaten eingegeben werden können, wobei diese Betriebsdaten zum Beispiel die Flüssigkeitsmenge wiedergeben, welche in das Chirurgieinstrument 20 zu fördern ist, wenn dieses bis zu seiner Auslassdüse 22 hin gefüllt werden soll.

Wenn also ein Chirurgieinstrument 20 an die Flüssigkeitsfördereinrichtung 10 angesteckt wird, so erzeugt der Ankoppelfühler 45 ein entsprechendes Signal, welches der Steuereinrichtung 30 mitgeteilt wird. Auf ein entsprechendes "Füllkommando" hin, welches der Operateur über die Eingabevorrichtung 32 eingibt, arbeitet nun die Flüssigkeitsfördereinrichtung 10 solange, bis das Chirurgieinstrument 20 bis zu seiner Auslassdüse 22 hin mit Schneidflüssigkeit gefüllt ist. Danach kann der Operateur das Gerät durch Betätigen der Bedienungstaste 23 aktivieren, also einen Strahl von Schneidflüssigkeit aus der Auslassdüse 22 derart austreten lassen, dass ein anvisiertes Zielgewebe durchtrennt wird. Die während der Operation abgegebene und tatsächlich aus der Auslassdüse 22 ausgetretene Flüssigkeitsmenge, also die Gesamtfördermenge abzüglich der im Chirurgieinstrument 20 enthaltenen Flüssigkeitsmenge wird auf der Anzeigeeinrichtung 31 angezeigt.

In Fig. 2 ist ein Druckverlauf idealisiert dargestellt. Der Druck p steigt nach diesem Diagramm an, während die Schneidflüssigkeit in die Anschlussleitung in Richtung auf die Auslassdüse 22 strömt, da das Luftpolster, welches die Flüssigkeit vor sich her treibt, immer kleiner wird. In dem Moment (t-Index 0), in welchem die Schneidflüssigkeit die Auslassdüse 22 erreicht, steigt der Druck abrupt an, da die Schneidflüssigkeit aufgrund ihrer gegenüber Luft sehr hohen Viskosität einen höheren Strömungswiderstand an der (sehr kleinen) Auslassdüse 22 aufweist. Dieser abrupte Druckanstieg kann dazu benützt werden, ein Signal abzugeben, welches anzeigt, dass nun das Chirurgieinstrument 20 korrekt gefüllt und somit betriebsbereit ist. Noch leichter ist die zweite Ableitung des Druckes nach der Zeit zum Generieren eines solchen Signals geeignet, da diese nur dann einen positiven Wert aufweist, wenn der genannte steile Druckanstieg tatsächlich vorliegt.

### Bezugszeichenliste

- 10: Flüssigkeitsfördereinrichtung
- 11: Vorratsbehälter
- 20: Chirurgieinstrument
- 21: Anschlussleitung
- 22: Auslassdüse
- 23: Bedienungstaste
- 30: Steuereinrichtung
- 31: Anzeigeeinheit
- 32: Eingabevorrichtung
- 41: Feuchtesensor
- 42: Drucksensor
- 43, 43': Positionssensor
- 45: Ankoppelfühler

## Patentansprüche

1. Wasserstrahlchirurgiegerät, umfassend
eine Flüssigkeitsfördereinrichtung (10), die zur Abgabe einer Flüssigkeit in eine Anschlussleitung (21) eines Chirurgieinstruments (20) mit einer Auslassdüse (22) durch Ansteuersignale aus einer Steuereinrichtung (30) ansteuerbar ist,
**dadurch gekennzeichnet, dass**
mindestens eine Messeinrichtung (41 - 43) vorgesehen und derart ausgebildet ist, dass nach einem Anschließen des Chirurgieinstruments (20) an die Flüssigkeitsfördereinrichtung (10) die Messeinrichtung (41 - 43) Messsignale zur Darstellung einer Menge tatsächlich aus der Auslassdüse (22) abgegebener Flüssigkeit in einer Anzeige- oder Registriereinheit (31) erzeugt.

2. Wasserstrahlchirurgiegerät nach Anspruch 1,
**dadurch gekennzeichnet, dass**
die Messeinrichtung (41, 42) einen Füllsensor umfasst und ein Füllsignal dann erzeugt, wenn die Anschlussleitung (21) im Wesentlichen bis zur Auslassdüse (22) gefüllt ist und eine zum Füllen der Anschlussleitung (21) benötigte Füllmenge wiedergibt.

3. Wasserstrahlchirurgiegerät nach einem der vorhergehenden Ansprüche, insbesondere nach Anspruch 2,
**dadurch gekennzeichnet, dass**
die Anzeige- oder Registriereinheit (31) derart ausgebildet ist, dass die Menge abgegebener Flüssigkeit abzüglich der Füllmenge angezeigt wird.

4. Wasserstrahlchirurgiegerät nach einem der vorhergehenden Ansprüche,
**dadurch gekennzeichnet, dass**
eine Eingabevorrichtung (32) vorgesehen ist, über welche der Steuereinrichtung (30) ein dem angeschlossenen Chirurgieinstrument (20) entsprechendes Mengensignal zur Eingabe einer von der Flüssigkeitsfördereinrichtung (10) abzugebenden Flüssigkeitsmenge zuführbar ist.

5. Wasserstrahlchirurgiegerät nach einem der vorhergehenden Ansprüche, insbesondere nach Anspruch 4,
**dadurch gekennzeichne**t, dass
die Eingabevorrichtung (32) manuell bedienbar ist.

6. Wasserstrahlchirurgiegerät nach einem der vorhergehenden Ansprüche, insbesondere nach Anspruch 4,
**dadurch gekenzeichnet**, dass
die Eingabevorrichtung (32) durch das Chirurgieinstrument (10) programmierbar ist.

7. Wasserstrahlchirurgiegerät nach einem der vorhergehenden Ansprüche, insbesondere nach Anspruch 2,
**dadurch gekennzeichnet, dass**
der Füllsensor einen Drucksensor (42) umfasst, der bei einer vorbestimmten Druckänderung oder einem vorbestimmten zeitlichen Verlauf des Druckes das Füllsignal erzeugt.

8. Wasserstrahlchirurgiegerät nach einem der vorhergehenden Ansprüche, insbesondere nach Anspruch 2,
**dadurch gekennzeichnet, dass**
die Sensoreinrichtung einen Feuchtigkeits- oder Leitfähigkeitssensor (41) umfasst.

## Claims

1. Water jet surgical device, comprising
a liquid conveying device (10) which is actuatable by means of actuation signals from a control device (30) so as to dispense a liquid into an attachment line (21) of a surgical instrument (20) with an outlet nozzle (22),
**characterized in that**
at least one measuring device (41 - 43) is provided and is designed such that, after the surgical instrument (20) has been attached to the liquid conveying device (10), the measuring device (41 - 43) generates measuring signals in order to show, on a display or registering unit (31), a quantity of liquid actually dispensed from the outlet nozzle (22) .

2. Water jet surgical device according to Claim 1,
**characterized in that**
the measuring device (41, 42) comprises a filling sensor and generates a filling signal when the attachment line (21) is filled substantially as far as the outlet nozzle (22) and represents a filling quantity required to fill the attachment line (21).

3. Water jet surgical device according to one of the preceding claims, in particular according to Claim 2,
**characterized in that**
the display or registering unit (31) is designed such that the quantity of dispensed liquid minus the filling quantity is displayed.

4. Water jet surgical device according to one of the preceding claims,
**characterized in that**
an input device (32) is provided by means of which a quantity signal corresponding to the connected surgical instrument (20) can be supplied to the control device (30) for the input of a liquid quantity that is to be dispensed by the liquid conveying device (10).

5. Water jet surgical device according to one of the preceding claims, in particular according to Claim 4,
**characterized in that**
the input device (32) is manually operable.

6. Water jet surgical device according to one of the preceding claims, in particular according to Claim 4,
**characterized in that**
the input device (32) is programmable by means of the surgical instrument (10).

7. Water jet surgical device according to one of the preceding claims, in particular according to Claim 2,
**characterized in that**
the filling sensor comprises a pressure sensor (42) which generates the filling signal in the presence of a predetermined pressure change or a predetermined profile with respect to time of the pressure.

8. Water jet surgical device according to one of the preceding claims, in particular according to Claim 2,
**characterized in that**
the sensor device comprises a moisture or conductivity sensor (41).

## Revendications

1. Appareil chirurgical à jet d'eau, comprenant
un système de transport de liquide (10), qui pour la distribution d'un liquide dans un conduit de raccordement (21) d'un instrument chirurgical (20) avec une buse de sortie (22) est activable par des signaux d'activation provenant d'un système de commande (30),
**caractérisé en ce**
**qu'**au moins un système de mesure (41 à 43) est prévu et conçu de telle sorte qu'après un raccordement de l'instrument chirurgical (20) sur le système de transport de liquide (10), le système de mesure (41 à 43) génère dans un module d'affichage ou d'enregistrement (31) des signaux de mesure représentant une quantité de liquide réellement distribuée hors de la buse de sortie (22) .

2. Appareil chirurgical à jet d'eau selon la revendication 1,
**caractérisé en ce que**
le système de mesure (41, 42) comprend un capteur de remplissage et génère un signal de remplissage lorsque le conduit de raccordement (21) est rempli sensiblement jusqu'à la buse de sortie (22) et reproduit une quantité de remplissage requise pour remplir le conduit de raccordement (21).

3. Appareil chirurgical à jet d'eau selon l'une quelconque des revendications précédentes, notamment selon la revendication 2,
**caractérisé en ce que**
le module d'affichage ou d'enregistrement (31) est conçu de telle sorte que la quantité de liquide distribué soit affichée en soustraction de la quantité de remplissage.

4. Appareil chirurgical à jet d'eau selon l'une quelconque des revendications précédentes,
**caractérisé en ce**
**qu'**il est prévu un dispositif de saisie (32) par l'intermédiaire duquel il peut être amené au système de commande (30) un signal de quantité correspondant à l'instrument chirurgical (20), destiné à saisir une quantité de liquide qui doit être distribuée par le système de transport de liquide (10).

5. Appareil chirurgical à jet d'eau selon l'une quelconque des revendications précédentes, notamment selon la revendication 4,
**caractérisé en ce que**
le dispositif de saisie (32) est actionnable manuellement.

6. Appareil chirurgical à jet d'eau selon l'une quelconque des revendications précédentes, notamment selon la revendication 4,
**caractérisé en ce que**
le dispositif de saisie (32) est programmable par l'instrument chirurgical (10).

7. Appareil chirurgical à jet d'eau selon l'une quelconque des revendications précédentes, notamment selon la revendication 2,
**caractérisé en ce que**
le capteur de remplissage comprend un capteur de pression (42), qui lors d'une variation prédéfinie de la pression ou dans le cas d'une courbe prédéfinie de la pression dans le temps génère le signal de remplissage.

8. Appareil chirurgical à jet d'eau selon l'une quelconque des revendications précédentes, notamment selon la revendication 2,
**caractérisé en ce que**
le système de capteurs comprend un capteur d'humidité ou de conductivité (41).
